# EUROPÄISCHE PATENTSCHRIFT

(11) **EP 0 490 985 B1**
(45) Veröffentlichungstag und Bekanntmachung des Hinweises auf die Patenterteilung: **12.01.1994**
(21) Anmeldenummer: 90914161.6
(22) Anmeldetag: 07.09.1990
(51) Int. Cl.: A61N 1/08

(54) **MEDIZINISCHES GERÄT ZUR STIMULATION VON GEWEBEKONTRAKTIONEN**
MEDICAL APPLIANCE FOR STIMULATING TISSUE CONTRACTIONS
APPAREIL MEDICAL POUR STIMULER LES CONTRACTIONS TISSULAIRES

(30) Priorität: 07.09.1989 DE 8911655 U
(43) Veröffentlichungstag der Anmeldung: 24.06.1992
(73) Patentinhaber: Pacesetter AB, 171 95 Solna (SE)
(72) Erfinder: HÖGNELID, Kurt, S-137 38 Västerhaninge (SE); STRANDBERG, Hans, S-172 36 Sundbyberg (SE); HOLMSTRÖM, Nils, S-175 45 Järfälla (SE)
(74) Vertreter: Lettström, Richard Wilhelm
(86) Internationale Anmeldenummer: EP9001516
(87) Internationale Veröffentlichungsnummer: WO9103272

(56) Entgegenhaltungen:
- EP-A- 236 562
- EP-A- 0 308 536
- FR-A- 2 524 808
- US-A- 4 726 379

## Beschreibung

Die Erfindung betrifft ein in den Körper eines Lebewesens inplantierbares medizinisches Gerät mit Mitteln zum elektrischen Stimulieren von Gewebekontraktionen, die einen ein Stimulationspotential und einen ein Bezugspotential führenden Anschluß aufweisen, mit Mitteln zum Detektieren stimulierter Gewebekontraktionen, die eingangsseitig zwei Anschlüsse aufweisen und ein dem Potentialunterschied zwischen den beiden Anschlüssen entsprechendes Signal bilden, mit Mitteln zum Anlegen des Bezugspotentials an den Körper des Lebewesens und mit einer bipolaren Elektrode, die zwei mit dem zu stimulierenden Gewebe in Kontakt bringbare Kontaktteile aufweist, wobei zur Stimulation einer Gewebekontraktion der das Stimulationspotential führende Anschluß der Mittel zum Stimulieren mit wenigstens einem der beiden Kontraktteile der Elektrode und der das Bezugspotential führende Anschluß der Mittel zum Stimulieren mit den Mitteln zum Anlegen des Bezugspotentials verbunden ist und nach erfolgter Stimulation einer der Anschlüsse der Mittel zum Detektieren stimulierter Gewebekontraktionen mit einem der beiden Kontaktteile der Elektrode verbunden ist.

Bei einem derartigen, aus der EP-A-O 236 562 bekannten medizinischen Gerät handelt es sich um einen Herzschrittmacher mit einer bipolaren Elektrode. Der bekannte Herzschrittmacher weist Mittel zum Stimulieren des Herzgewebes auf, die ausgangsseitig über einen das Stimulationspotential führenden Anschluß mit einem ersten Kontaktteil am distalen Ende der Elektrode und über einen ein Bezugspotential führenden Anschluß mit dem Gehäuse des Herzschrittmachers verbunden sind. Zum Detektieren der Reaktion des Herzens auf die Stimulation (Stimulationsantwort) sind entsprechende Mittel eingangsseitig mit dem Bezugspotential einerseits und einem zweiten ringförmigen Kontaktteil der bipolaren Elektrode andererseits verbunden. Dadurch, daß das mit dem Stimulationspotential beaufschlagte erste Kontaktteil und das die Stimulationsantwort erfassende zweite Kontaktteil vorzugsweise mindestens 0,5 cm voneinander beabstandet angeordnet sind, wird die Überlagerung der detektierten Stimulationsantwort durch die bei der vorangegangenen Stimulation im Gewebebereich um das erste Kontaktteil herum hervorgerufenen Polarisationserscheinungen reduziert.

Gemäß der Erfindung ist vorgesehen, daß bei dem medizinischen Gerät der eingangs angegebenen Art nach erfolgter Stimulation der andere Anschluß der Mittel zum Detektieren stimulierter Gewebekontraktionen mit dem anderen Kontakteil der Elektrode verbunden ist. Die stimulierte Gewebekontraktion wird also bipolar zwischen den beiden Kontaktteilen der Elektrode erfaßt. Es ist zwar u.a. aus der USA-4 532 931, US-A-4 726 379 und FR-A-2 524 808 bekannt, Gewebekontraktionen bipolar zu detektieren, jedoch handelt es sich bei den bisher bekannten Beispielen nur um die Detektion spontaner, nicht jedoch stimulierter Gewebekontraktionen. Infolge des Umstandes, daß bei dem erfindungsgemäßen Gerät beide Kontaktteile mit dem zu stimulierenden Gewebebereich in Kontakt stehen, wird das Gewebe nach einer Stimulation infolge der damit verbunden Polarisation im Bereich beider Kontaktteile etwa das gleiche elektrische Potential aufweisen. Da die Mittel zum Detektieren stimulierter Gewebekontraktionen ein dem Potentialunterschied zwischen ihren Anschlüssen entsprechendes Signal bilden und ihre Anschlüsse im Anschluß an die Stimulation mit jeweils einem Kontaktteil verbunden sind, kann die Polarisation des Gewebes also zu keinem nennenswerten Ausgangssignal der Mittel zum Detektieren stimulierter Gewebekontraktionen führen, was bedeutet, daß eine Übersteuerung der Mittel zum Detektieren stimulierter Gewebekontraktionen ausgeschlossen ist. Löst eine Stimulation eine Gewebekontraktion aus, tritt infolge des Umstandes, daß sich die mit der stimulierten Gewebekontraktion verbundene Potentialänderung des Gewebes mit endlicher Geschwindigkeit fortpflanzt, die Potentialänderung an einem der Kontaktteile früher als an dem anderen auf, so daß eine Potentialdifferenz zwischen den beiden Kontaktteilen und damit den Anschlüssen der Mittel zum Detektieren stimulierter Gewebekontraktionen auftritt, diese also ein der Detektion einer stimulierten Gewebekontraktion entsprechendes Signal liefern. Der Abstand zwischen den Kontaktteilen sollte aber einen Millimeter nicht unterschreiten, um sicherzustellen, daß die Zeitdifferenz, mit der die mit einer stimulierten Gewebekontraktion verbundene Potentialänderung an den Kontaktteilen auftritt, ausreichend groß ist, um eine Detektion zu ermöglichen. Es wird somit deutlich, daß mittels des erfindungsgemäßen Gerätes eine sichere Detektion stimulierter Gewebekontraktionen auf technisch einfache, kostengünstige und energiesparende Weise möglich ist.

Gemäß einer ersten bevorzugten Variante der Erfindung ist vorgesehen, daß zur Stimulation einer Gewebekontraktion der das Stimulationspotential führende Anschluß der Mittel zum Stimulieren mit nur einem Kontaktteil der Elektrode verbunden ist und daß der Abstand zwischen den Kontaktteilen der Elektrode höchstens fünf Millimeter beträgt. Durch diese Maßnahme ist, obwohl nur eines der Kontaktteile der Elektrode mit dem das Stimulationspotential führenden Anschluß verbunden ist, sichergestellt, daß nach erfolgter Stimulation der zu stimulierende Gewebebereich im Bereich beider Kontaktteile der Elektrode etwa im gleichen Maße polarisiert ist, da der Abstand der Kontaktteile höchstens fünf Millimeter beträgt. Übersteuerungen der Mittel zum Detektieren stimulierter Gewebekontraktionen sind somit ausgeschlossen.

Eine weitere bevorzugte Ausführungsform der Erfindung sieht vor, daß Schaltmittel vorgesehen sind, die zur Stimulation einer Gewebekontraktion den das Stimulationspotential führenden Anschluß der Mittel zum Stimulieren mit beiden Kontaktteilen der Elektrode verbinden. Infolge dieser Maßnahme liegt nach einer Stimulation im Bereich beider Kontaktteile exakt die gleiche Gewebepolarisation vor, da der zu stimulierenade Gewebeberich im Bereich beider Kontaktteile mit der Stimulationsenergie beaufschlagt wird. Die Kontaktteile können somit auch einen Abstand voneinander aufweisen, der fünf Millimeter übersteigt. Allerdings muß gewährleistet sein, daß beide Kontaktteile noch mit dem zu stimulierenden Gewebebereich in Kontakt stehen, um zu vermeiden, daß auch Gewebereiche, die an sich nicht stimuliert werden sollen, eine Stimulation erfahren.

Eine sichere Detektion der stimulierten Gewebekontraktionen wird in vorteilhafter Weise dadurch erreicht, daß die Mittel zum Detektieren stimulierter Gewebekontraktionen einen Bandpaßfilter mit einem nachgeordneten Schwellenwertdetektor enthalten. Dabei ist die Übertragungsfunktion des Bandpaßfilters derart gewählt, daß nur diejenigen Signalanteile, die hinsichtlich ihrer Frequenz oder Steilheit für stimulierte Gewebekontraktionen typisch sind, das Bandpaßfilter passieren können. Entspechend einer vorteihaften Weiterbildung der Erfindung ist vorgesehen, daß außerdem Mittel zum Detektieren spontaner Gewebekontraktionen vorgesehen und mit einer Steuerlogik verbunden sind, die Mittel zum Stimulieren jeweils dann aktiviert, wenn mittels der Mittel zum Detektieren spontaner Gewebekontraktionen vor Ablauf eines definierten Zeitintervalls keine spontane Gewebekontraktion detektierbar ist. Stimulationen von Gewebekontraktionen ausbleiben und Stimulationen tatsächlich erforderlich sind.

Dabei müssen nicht unbedingt separate Mittel zum Detektieren spontaner Gewebekontraktionen vorhanden sein, da die Mittel zum Detektieren spontaner Gewebekontraktionen gemäß einer bevorzugten Variante der Erfindung durch die Mittel zum Detektieren stimulierter Gewebekontraktionen gebildet sein können, wobei ferner Mittel zum Einstellen unterschiedlicher Empfindlichkeiten der Mittel zum Detektieren stimulierter bzw. spontaner Gewebekontraktionen vorgesehen sind. Um eine unipolare Detektion spontaner Gewebekontraktionen zu ermöglichen, ist es in diesem Falle zweckmäßig, wenn Schaltmittel vorgesehen sind, mittels derer zum Detektieren spontaner Gewebekontraktionen ein Anschluß der Mittel zum Detektieren stimulierter Gewebekontraktionen mit den Mitteln zum Anlegen des Bezugspotentials verbindbar ist.

Vorzugsweise sind jedoch die Mittel zum Detektieren sowohl stimulierter als auch spontaner Gewebekontraktionen eingangsseitig fest mit den beiden Kontaktteilen der Elektrode verbunden. Das bedeutet, daß keine Schaltmittel benötigt werden und daß die Detektion sowohl der stimulierten als auch der spontanen Gewebekontraktionen mit denselben Mitteln bipolar erfolgt. Auf diese Weise ist es auch einfacher, eventuelle Störsignale zu erfassen und zu eliminieren, weil die Detektion der stimulierten und spontanen Gewebekontraktionen kontinuierlich mit denselben Mitteln erfolgt. In diesem Zusammennang wird eine sichere Detektion dadurch erreicht, daß die Mittel zum elektischen Stimulieren von Gewebekontrationen ein gegenüber dem Bezugspotential negatives Stimulationspotential erzeugen, daß nach einer erfolgten Stimulation zur Detektion stimulierter Gewebekontraktionen die Mittel zum Einstellen unterschiedlicher Detektionsempfindlichkeiten einen ersten Schwellenwert an dem Schwellenwertdetektor einstellen, der das bandpaßgefilterte Signal auf ein Überschreiten des ersten Stellenwertes überwacht, und daß in einem nachfolgenden zweiten Zeitintervall zur Detektion spontaner Gewebekontraktionen die Mittel zum Einstellen unterschiedlicher Detektionsempfindlichkeiten einen zweiten Schwellenwert an dem Schwellenwertdetektor einstellen, der das bandpaßgefilterte Signal auf ein Unterschreiten des zweiten Schwellenwertes überwacht. Dabei wird in vorteilhafter Weise der Umstand ausgenutzt, daß bei dem der stimulierten Gewebekontraktion entsprechenden Signal die positve Signalflanke und bei einer natürlichen Gewebekontraktion die negative Signalflanke jeweils am steilsten und somit für eine sichere Detektion am besten geeignet ist.

Zur Erläuterung der Erfindung wird im folgenden auf die Figuren der Zeichnung Bezug genommen; im einzelnen zeigen:
- Fig. 1: ein erstes Ausführungsbeispiel des als Herzschrittmacher ausgeführten erfindungsgemäßen Gerätes,
- Fig. 2: in vergrößerter Darstellung das zur Verankerung im Herzen vorgesehene Ende der Elektrode des Herzschrittmachers,
- Fig. 3: ein zweites und
- Fig. 4: ein drittes Ausführungsbeispiel für den Herzschrittmacher,
- Fig. 5: ein Blockschaltbild des Stimulationsimpuls-Generators und der Detektoreinrichtung zur Detektion stimulierter Herzmuskelkontraktionen,
- Fig 6: ein Zeitdiagramm mit dem von dem Stimulationsimpuls-Generator erzeugten Stimulationsimpuls und Steuersignalen für die in Fig. 5 dargestellten Schaltungsteile und
- Fig.7: Verläufe von stimulierten und spontanen Herzmuskelkontraktionen vor und nach einer Bandpaßfilterung

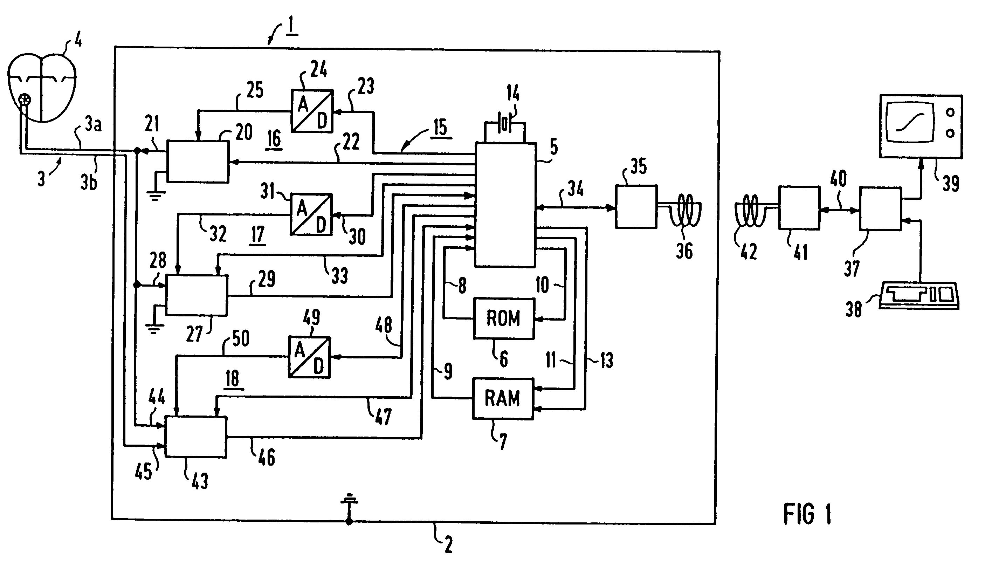

Bei dem in Fig. 1 gezeigten Ausführungsbeispiel ist mit 1 ein implantierbarer Herzschrittmacher bezeichnet, dessen Bauteile in einem schematisch angedeuteten, hermetisch dichten, implantierbaren Gehäuse 2 aus einem elektrisch leitenden Werkstoff, z.B. Titan, aufgenommen sind. Von dem im VVI-Mode arbeitenden Herzschrittmacher 1 führt eine bipolare Elektrode 3 zu einem schematisch angedeuteten Herz 4 eines Lebewesens und ist dort in ein Ventrikel, vorzugsweise das rechte, eingepflanzt. Die bipolare Elektrode 3 besitzt zwei Leiter 3a, 3b.

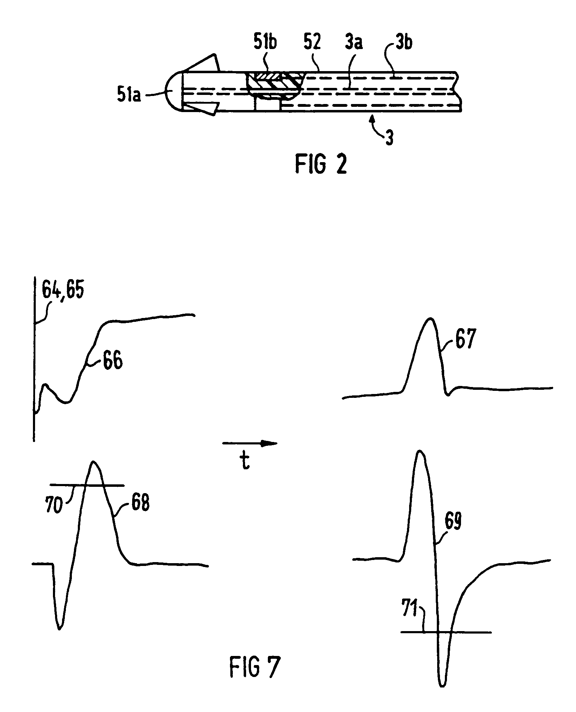

Wie aus der Fig. 2 ersichtlich ist, besteht die bipolare Elektrode 3 aus einem Koaxialkabel 52, dessen Innenleiter der Leitung 3a und dessen Abschirmung der Leitung 3b entspricht. Die Leitungen 3a und 3b führen zu zwei Kontaktteilen 51a und 51b, die dazu dienen, das Herzmuskelgewebe elektrisch zu kontaktieren. Das Kontaktteil 51a ist als Spitze ausgebildet, während das Kontaktteil 51b als Ring ausgeführt ist. Zwischen den beiden Kontaktteilen 51a, 51b liegt ein Abstand in der Größenordnung von fünf Millimetern.

Der Herzschrittmacher 1 umfaßt u.a. einen Mikroprozessor 5, dem ein Nur-Lese-Speicher (ROM) 6 und ein Schreib-Lese-Speicher (RAM) 7 zugeordnet sind, die über Datenleitungen 8, 9 und Adreßleitungen 10, 11 mit dem Mikroprozessor 5 in Verbindung stehen. Zu dem RAM 7 führt von dem Mikroprozessor 5 außerdem eine Leitung 13, die zum Umschalten des RAM 7 von Schreib- auf Lesebetrieb und umgekehrt dient. In dem ROM 6 ist ein Programm gespeichert, mittels dessen sämtliche Funktionen des Herzschrittmachers 1 gesteuert werden. Wenn also im folgenden die Rede davon ist, daß der Mikroprozessor 5 eine bestimmte Funktion ausführt, so ist darunter zu verstehen, daß der Mikroprozessor 5 durch Ausführung des im ROM 6 gespeicherten Programms unter Heranziehung von in dem RAM 7 befindlichen Daten und ihm anderweitig, z.B. über eine Eingangs-Beschaltung, zugeführten Daten zur Ausführung der jeweiligen Funktion tätig wird. Wenn die Rede davon ist, daß der Mikroprozessor 5 für einen Parameter einen bestimmten Wert einstellt, so bedeutet dies, wenn nichts anderes angegeben ist, daß dem bestimmten Wert entsprechende Daten in dem RAM 7 gespeichert sind, auf die der Mikroprozessor 5 zugreifen kann.

Mit dem Mikroprozessor 5 ist ein Quarzkristall 14 verbunden, der zur Erzeugung der für den Betrieb des Mikroprozessors 5 erforderlichen Taktsignale dient und außerdem die Zeitreferenz für den Betrieb des Herzschrittmachers 1 darstellt. Der Mikroprozessor 5 besitzt eine insgesamt mit 15 bezeichnete Eingangs/Ausgangs-Beschaltung, die mehrere Kanäle 16,17,18 aufweist.

Der Kanal 16 dient dazu, das Herz 4 erforderlichenfalls mit elektrischen Stimulationsimpulsen zur Auslösung von stimulierten Herzmuskelkontraktionen zu versorgen. Der Kanal 16 besitzt daher einen Stimulationsimpuls-Generator 20, der einen mittels einer Ausgangsleitung 21 mit der Leitung 3a der Elektrode 3 verbundenen und das Stimulationspotential führenden Anschluß sowie einen ein Bezugspotential führenden Anschluß aufweist. Der das Bezugspotential führende Anschluß ist mit dem Gehäuse 2 elektrisch leitend verbunden. Dies ist dadurch veranschaulicht, daß sowohl der das Bezugspotential führende Anschluß des Stimulationsimpuls-Generators 20 als auch das Gehäuse 2 mit einem Massesymbol versehen sind. Obwohl eine bipolare Elektrode 3 vorhanden ist, erfolgt also die Stimulation unipolar, d.h., daß das Stimulationspotential über die Spitze 51a der Elektrode 3 an das Herzmuskelgewebe angelegt wird und das Bezugspotential außerhalb des Herzens an das das Gehäuse 2 des Herzschrittmachers 1 umgebende Gewebe angelegt wird. Der Stimulationsimpuls-Generator 20 ist über eine Leitung 22, die mit einem entsprechenden Ausgang des Mikroprozessors 5 verbunden ist, zur Abgabe eines Stimulationsimpulses aktivierbar. Digitale Daten, die die Dauer und die Amplitude und damit den Energiegehalt der Stimulationsimpulse betreffen, gelangen von dem Mikroprozessor 5 über eine Leitung 23 zu einer Digital/Analog-Schnittstelle 24, die dem Stimulationsimpuls-Generator 20 über eine Steuerleitung 25 den digitalen Daten entsprechende analoge Steuersignale zuführt. Diese stellen den Stimulationsimpuls-Generator 20 derart ein, daß er bei Bedarf Stimulationsimpulse eines bestimmten Energiegehaltes erzeugt und somit das Herz 4 mit einer bestimmten Stimulationsintensität stimuliert.

Der Kanal 17 besitzt eine erste Detektoreinrichtung 27, die ausschließlich dazu dient, spontane Herzmuskelkontraktionen, also natürliche Herzschläge, zu detektieren. Die erste Detektoreinrichtung 27 besitzt einen mit der Leitung 3a der Elektrode 3 über eine Eingangsleitung 28 verbundenen Signaleingang und einen ein Bezugspotential führenden Anschluß. Dabei handelt es sich bei dem Bezugspotential, wie durch das an dem das Bezugspotential führenden Anschluß angebracht Massesymbol deutlich wird, um das gleiche Bezugspotential, das auch der entsprechende Anschluß des Stimulationsimpuls-Generators 20 und das Gehäuse 2 führen. Obwohl eine bipolare Elektrode 3 vorhanden ist, erfolgt also bei dem Ausführungsbeispiel nach Fig. 1 auch die Detektion spontaner Herzmuskelkontraktionen mittels der ersten Detektoreinrichtung 27 unipolar. Das der elektrischen Aktivität des Herzens 4 entsprechende Signal wird somit mittels der Spitze 51a der Elektrode 3 abgegriffen und der das Bezungspotential führende Anschluß der ersten Detektoreinrichtung 27 liegt auf dem Potential des das Gehäuse 2 des Herzschrittmachers 1 umgebenden Gewebes. Detektiert die zweite Detektoreinrichtung 27 in dem ihr über die Leitung 3a der Elektrode 3 zugeführten, der elektrischen Aktivität des Herzens 4 entsprechenden Signal eine spontane Herzmuskelkontraktion, gibt sie über eine Leitung 29 ein dies anzeigendes Signal an einen entsprechenden Eingang des Mikroprozessors 5. Dieses Signal gibt die erste Detektoreinrichtung 27 dann ab, wenn in dem der elektrischen Aktivität des Herzens entsprechenden Signal ein Ereignis mit für einen natürlichen Herzschlag typischer Steilheit und/oder Amplitude auftritt. Der Mikroprozessor 5 ist über eine Leitung 30 mit einer Digital/Analog-Schnittstelle 31 verbunden, die ihr von dem Mikroprozessor 5 zugeführte digitale Daten als entsprechende analoge Signale über eine Steuerleitung 32 an die erste Detektoreinrichtung 27 weitergibt. Die digitalen Daten bzw. die entspechenden analogen Signale diene dazu, die Empfindlichkeit der zweiten Detektoreinrichtung 27, also diejenige Steilheit und/oder Amplitude, einzustellen, die ein Ereignis in dem der elektrischen Aktivität des Herzens 4 entsprechenden Signal wenigstens aufweisen muß, um als natürlicher Herzschlag detektiert zu werden. Über eine Steuerleitung 33 kann der Mikroprozessor 5 außerdem der ersten Detektoreinrichtung 27 ein Signal zuführen, das diese völlig sperrt, so daß keine auf die Detektion eines natürlichen Herzschlages hinweisenden Signale zu dem Mikroprozessor 5 gelangen können.

Erhält der Mikroprozessor 5 über die Leitung 29 ein die Detektion eines natürlichen Herzschlages mittels der ersten Detektoreinrichtung 27 anzeigendes Signal oder aktiviert er über die Leitung 22 den Stimulationsimpuls-Generator 20 zur Abgabe eines Stimulationsimpulses, beginnt der Mikroprozessor 5 als Zähler zu arbeiten und zählt eine Anzahl von aus der Schwingung des Quarzes 14 abgeleiteten Taktimpulsen ab, die einem Basis-Zeitintervall entspricht. Das Basis-Zeitintervall bestimmt diejenige Stimulationsfrequenz, mit der das Herz 4 beim Ausbleiben natürlicher Herzschläge stimuliert wird. Gelangt während eines Basis-Zeitintervalls über den Kanal 17, d.h. über die Leitung 29, kein Signal zu dem Mikroprozessor 5, das die Detektion eines natürlichen Herzschlages anzeigt, aktiviert der Mikroprozessor 5 bei Ablauf des Basis-Zeitintervalls über die Leitung 22 den Stimulationsimpuls-Generator 20. Im Anschluß an die Abgabe eines Stimulationsimpulses beginnt der Mikroprozessor 5 erneut eine Anzahl von Taktimpulsen abzuzählen, die dem die Stimulationsfrequenz bestimmenden Basis-Zeitintervall entspricht. Erhält der Mikroprozessor 5 dagegen während des Laufes des Basis-Zeitintervalls ein die Detektion eines natürlichen Herzschlages anzeigendes Signal von der ersten Detektoreinrichtung 27, bricht er den beschriebenen Zählvorgang ab, sofern ein weiteres Zeitintervall, die sogenannte Refraktärzeit abgelaufen ist, und beginnt den beschriebenen Zählvorgang zur Bestimmung des Basis-Zeitintervalls von neuem, ohne daß die Abgabe eines Stimulationsimpulses erfolgt. Die Detektion eines natürlichen Herzschlages mittels der ersten Detektoreinrichtung 27 nach Ablauf der Refraktärzeit inhibiert also die Abgabe eines Stimulationsimpulses. Die Refraktärzeit, die grundsätzlich kürzer ist als das beispielsweise auf Werte zwischen 400 und 2000 ms programmierbare Basis-Zeitintervall, dauert etwa zwischen 250 und 450 ms (programmierbar). Die Refraktärzeit ist in eine absolute Refraktärzeit und eine sich an diese anschließende relative Refraktärzeit unterteilt, wobei während der absoluten Refraktärzeit die erste Detektoreinrichtung 27 völlig gesperrt ist.

Der Mikroprozessor 5 ist über eine Leitung 34 mit einem Telemetrieschaltkreis 35 verbunden, an den eine Sende/Empfangs-Spule 36 angeschlossen ist. Der Herzschrittmacher 1 ist somit in der Lage, mit einem externen Programmiergerät 37, an das eine Tastatur 38 und einen Monitor 39 angeschlossen sind, Daten auszutauschen, da das Programmiergerät 37 über eine Leitung 40 mit einem zweiten Telemetrieschaltkreis 41 verbunden ist, an den wieder eine Sende/Empfangs-Spule 42 angeschlossen ist. Zum Datenaustausch zwischen dem Herzschrittmacher 1 und dem Programmiergerät 37 wird die Sende/Empfangs-Spule 42 des zu dem Programmierrgerät 37 gehörigen Telemetrieschaltkreises 41 so auf der Körperoberfläche des den Herzschrittmacher 1 tragenden Lebewesens positioniert, daß sie mit der Sende/ Empfangs-Spule 36 des Herzschrittmachers 1 induktiv gekoppelt ist. Es besteht dann die Möglichkeit, dem Programmiergerät 27 die in dem ROM 6 und dem RAM 7 befindlichen Daten zur Überprüfung zuzuführen. Außerdem besteht die Möglichkeit, dem RAM 7 des Herzschrittmachers 1 von dem Programmiergerät 37 her veränderte bzw. zusätzliche Daten zuzuführen, die das Betriebsverhalten des Herzschrittmachers 1, also dessen Zusammenspiel mit dem zu stimulierenden Herz 4, beeinflussen und verändern. Dieser Vorgang wird gewöhnlich als Programmierung bezeichnet.

Der Kanal 18 der Eingangs/Ausgangs-Beschaltung des Mikroprozessors 5 dient dazu, diesem Daten zur Verfügung zu stellen, die es diesem anhand des in dem ROM 6 gespeicherten Programms gestatten, die Stimulationsintensität, also den Energiegehalt der mittels des Stimulationsimpuls-Generators 20 erzeugten Stimulationsimpulse, so einzustellen, daß ein Stimulationsimpuls auch tatsächlich eine Herzmuskelkontraktion, also eine stimulierten Herschlag, auslöst. Der Kanal 18 enthält zu diesem Zweck eine zweite Detektoreinrichtung 43, die ausschließlich zur Detektion stimulierter Herzmuskelkontraktionen vorgesehen ist. Die zweite Detektoreinrichtung besitzt zwei Anschlüsse, von denen der eine über die Leitung 44 mit der Leitung 3a der Elektrode 3 und damit mit der Spitze 51a und der andere über eine Leitung 45 mit der Leitung 3b der Elektrode 3 und damit mit dem Ring 51b verbunden ist. Die zweite Detektoreinrichtung 43 detektiert im Gegensatz zu der bereits beschriebenen ersten Detektoreinrichtung 27 also Herzmuskelkontraktionen, die auf einen durch den Stimulationsimpuls-Generator 20 abgegebenen Stimulationsimpuls hin auftreten. Detektiert die zweite Detektoreinrichtung 43 eine stimulierte Herzmuskelkontraktion, gelangt ein entsprechendes Signal über eine Leitung 46 vom Ausgang der zweiten Detektoreinrichtung 43 zu einem entsprechenden Eingang des Mikropzessors 5. Der Mikroprozessor 5 ist in der Lage, über eine Steuerleitung 47 durch ein entsprechendes Signal den Eingang oder Ausgang der zweiten Detektoreinrichtung 43 zu sperren. Die Empfindlichkeit der zweiten Detektoreinrichtung 43 ist mittels des Mikroprozessors 5 in noch näher zu beschreibender Weise einstellbar, indem der Mikroprozessor 5 über eine Leitung 48 einer Digital/Analog-Schnittstelle 49 digitale Daten zuführt, die diese in ein entsprechendes analoges Signal umsetzt, das der zweiten Detektoreinrichtung 43 über eine Steuerleitung 50 zugeführt ist.

Anders als im Falle der ersten Detektoreinrichtung 27, deren Ausgang der Mikroprozessor 5 nur nach Ablauf der absoluten Refraktärzeit freischaltet, müssen im Falle der zweiten Detektoreinrichtung 43 Detektionen unmittelbar nach einer Stimulation erfolgen können, um die Detektion stimulierter Herzmuskelkontraktionen überhaupt zu ermöglichen. Der Mikroprozessor 5 gibt also die zweite Detektoreinrichtung 43 einige Millisekunden nach der Abgabe eines Stimulationsimpulses für ein kurzes Zeitintervall, z.B. 100 ms, zur Detektion stimulierter Herzmuskelkontraktionen frei. Dabei ist jedoch wesentlich, daß die Detektion einer stimulierten Herzmuskelkontraktion mittels der zweiten Detektoreinrichtung 43 die Abgabe eines Stimulationsimpulses mittels des Stimulationsimpuls-Generators 20 nicht inhibieren kann. Dies kann nur durch die Detektion eines natürlichen Herzschlages mittels der ersten Detektoreinrichtung 27 unter den beschriebenen Voraussetzungen erfolgen.

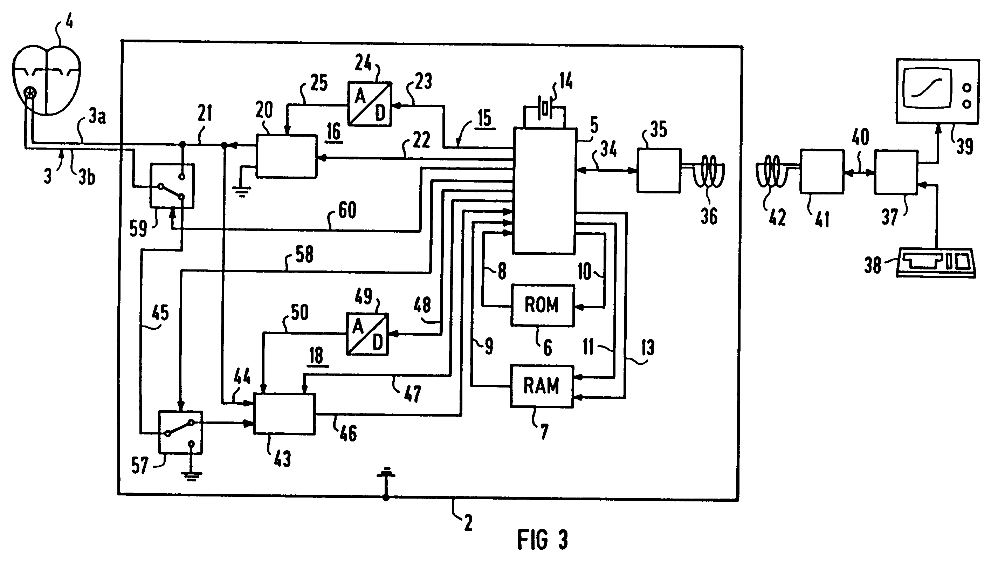

In der Fig. 3 ist ein zweites Ausführungsbeispiel für den erfindungsgemäßen Herzschrittmacher dargestellt, das sich von dem zuvor beschriebenen nur in einigen Punkten unterscheidet, weshalb gleiche Elemente jeweils die gleichen Bezugszeichen tragen. Der Herzschrittmacher gemäß Fig. 3 unterscheidet sich von dem zuvor beschriebenen zunächst dadurch, daß der Kanal 17 mit der Detektoreinrichtung 27 fehlt. Demzufolge dient die Detektoreinrichtung 43 des Kanals 18 nicht nur zur Detektion stimulierter Herzmuskelkontraktionen, sondern auch zur Detektion spontaner Herzmuskelkontraktionen. Der Mikroprozessor 5 gibt daher den Eingang bzw. Ausgang der Detektoreinrichtung 43 über die Leitung 47 nicht nur in der zuvor beschriebenen Weise im Anschluß an die Abgabe eines Stimulationsimpulses frei, sondern außerdem auch während jedes Basis-Zeitintervalls im Anschluß an die absolute Refraktärzeit, was erforderlich ist, um die Detektion spontaner Herzmuskelkontraktionen mittels der Detektoreinrichtung 43 zu ermöglichen. Dabei kann vorgesehen sein, daß der Mikroprozessor 5 über die Digital/Analog-Schnittstelle 49 unterschiedliche Empfindlichkeiten der Detektoreinrichtung 43 zum Detektieren stimulierter bzw. spontaner Herzmuskelkontraktionen einstellt. Wie aus der Fig 3 ersichlich ist, liegt in der von der mit dem Ring 51b der Elektrode 3 verbundenen Leitung 3b zu der Detektoreinrichtung 43 führenden Leitung 45 ein elektronischer Umschalter 57, der mittels des Mikroprozessors 5 über eine Steuerleitung 58 betätigbar ist. Dabei nimmt der Umschalter 57 zur Detektion stimulierter Herzmuskelkontraktionen die in Fig. 3 dargestellte Schaltstellung ein, in der die Eingänge der Detektoreinrichtung 43 in der im Zusammenhang mit der Fig. 1 beschriebenen Weise mit der Spitze 51a bzw. dem Ring 51b der Elekrode 3 verbunden sind. Zur Detektion spontaner Herzmuskelkontraktionen bringt der Mikroprozessor 5 den Umschalter 57 in seine nicht dargestellte Schaltstellung, in der die Leitung 45 mit dem Bezugspotential und damit mit dem Gehäuse 2 des Herzschrittmachers 1 verbunden ist; die Detektion spontaner Herzmuskelkontraktionen erfolgt also unipolar.

Ein weiter Unterschied besteht darin, daß der Herzschrittmacher gemäß Fig 3 einen weiteren elektronischen Umschalter 59 enthält, der von dem Mikroprozessor 5 über die Steuerleitung 60 betätigt wird. Nimmt der elektronische Umschalter 59 seine in Fig. 3 gezeigte Schaltstellung ein, ist die Leitung 3b der Elektrode 3 mit der Leitung 45 verbunden, so wie dies zur Detektion stimulierter und spontaner Herzmuskelkontraktionen erforderlich ist. Für die Abgabe eines Stimulationsimpulses betätigt der Mikroprozessor 5 den Umschalter 59 jedoch derart, daß dieser seine in Fig. 3 nicht gezeigte Schaltstellung einnimmt, in der die Leitung 3b der Elektrode 3 mit der von dem das Stimulationspotential führenden Anschluß des Stimulationsimpuls-Generators 20 zu der Leitung 3a der Elektrode 3 führenden Leitung 21 verbunden ist. Dabei ist wesentlich, daß der Mikroprozessor 5 die Schaltstellung des Umschalters 59 nur für die Dauer des Stimulationsimpulses in der beschriebenen Weise ändert. Es wird also deutlich, daß dem Herzmuskelgewebe ein Stimulationsimpuls sowohl über die Spitze 51a als auch den Ring 51b der Elektrode 3 zugeführt wird. Demzufolge weist das Herzmuskelgewebe nach der Abgabe eines Stimulationsimpulses sowohl im Bereich der Spitze 51b als auch im Bereich des Ringes 51b der Elektrode 3 jeweils die gleiche Polarisation auf. Demzufolge kann der zwischen der Spitze 51a und dem Ring 51b der Elektrode 3 vorliegender Abstand auch größer als fünf Millimeter sein, ohne daß die Gefahr einer Übersteuerung der Detektoreinrichtung 43 besteht. Sofern der Abstand kleiner als fünf Millimeter ist, kann der weitere Umschalter 59 auch entfallen, ebenso wie der weitere Umschalter 59 bei dem Ausführungsbeispiel nach Fig. 1 vorgesehen werden kann, wenn dort der Abstand zwischen den Kontaktteilen 51a, 51b größer als fünf Millimeter ist.

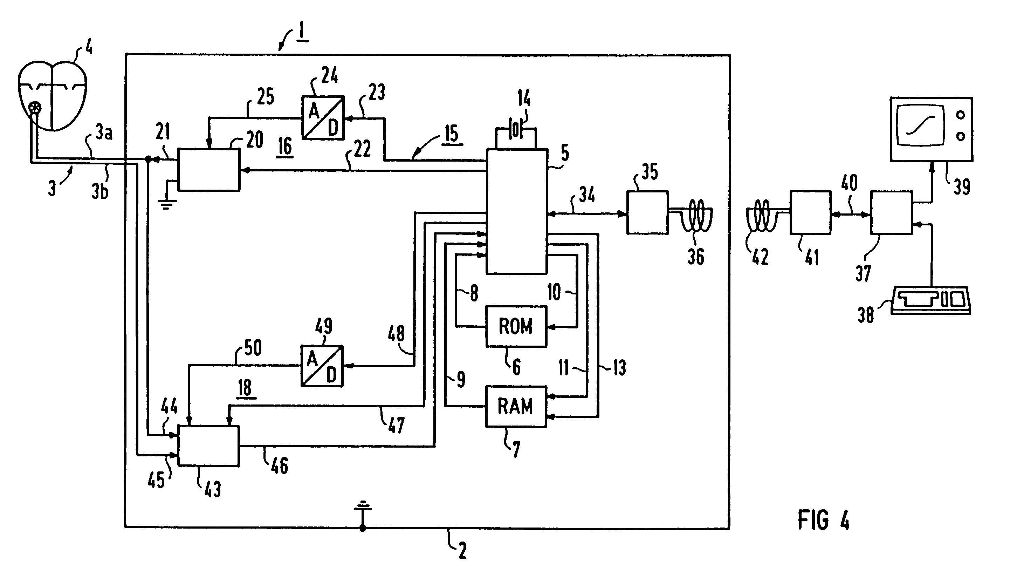

In Fig. 4 ist ein drittes Ausführungsbeispiel für den erfindungsgemäßen Herzschrittmacher dargestellt, bei dem ebenso wie bei dem Ausführungsbeispiel nach Fig. 3 die Detektoreinrichtung 43 sowohl zur Detektion sponanter als auch stimulierter Herzmuskelkontraktionen dient. Im Unterschied zu dem zuvor beschriebenen Ausführungsbeispiel ist jedoch die Detekoreinrichtung 43 mit ihren eingangsseitigen Anschlüssen fest mit den Kontaktteilen 51a, 51b der Elektrode 3 verbunden, so daß die Detektion sowohl der spontanen als auch der stimulierten Herzmuskelkontraktionen bipolar erfolgt. Die Detektoreinrichtung 43 ist nach jedem Stimulationsimpuls für ein vorgegebenes Zeitintervall zur Detektion einer möglichen stimulierten Herzmuskelkontraktion aktiviert und anschließend nach Ablauf der Refraktärzeit zur Detektion von natürlichen Herzschlägen. Dabei kann der Mikroprozessor 5 über die Digital/Analog-Schnittstelle 49 unterschiedliche Empfindlichkeiten der Detektoreinrichtung für die Detektion stimulierter bzw. spontaner Herzmuskelkontraktonen einstellen.

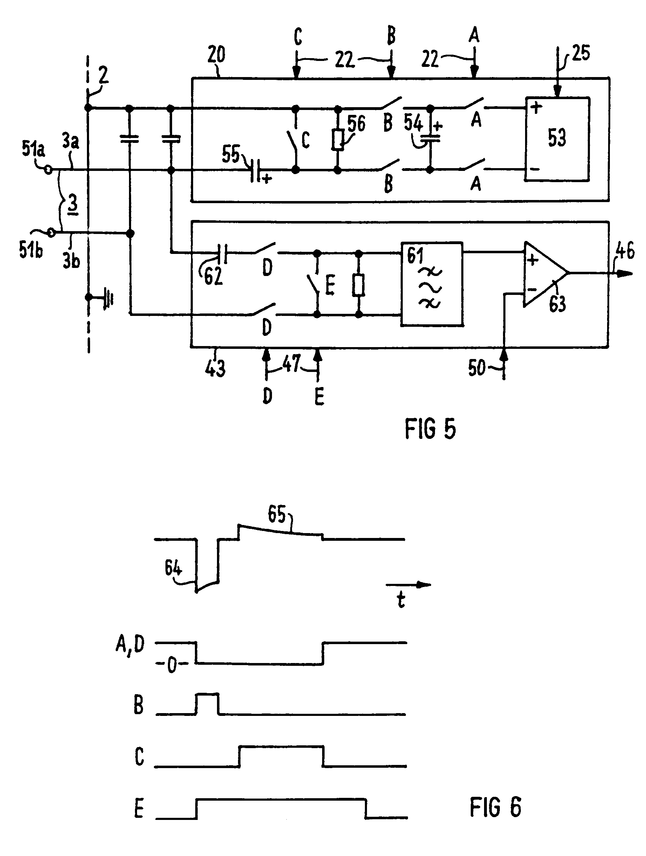

In Figur 5 sind der in den unterschiedlichen Ausführungsbeispielen angegebene Stimulationsimpuls- Generator 20 und die Detektoreinrichtung 43 entsprechend der Verschaltung nach Fig. 4 näher dargestellt. Der Stimulationsimpuls-Generator 20 enthält eine batteriegespeiste Ladeschaltung 53 mit der ein über ein erstes Schalterpaar A an dem Ausgang der Ladeschaltung 53 angeschlossener Ladekondensator 54 auf einen durch den Mikroprozessor 5 über die Digital-/Analog-Schnittstelle 24 vorgegebenen Spannungswert aufgeladen wird. Der Ladekondensator 54, ist über ein zweites Schalterpaar B an seinem ein positives Bezugpotential führenden Anschluß mit dem Gehäuse 2 des Herzschrittmachers 1 und an seinem das negative Stimulationspotential führenden Anschluß über einen Ausgangskondensator 55 mit dem Kontaktteil 51a an der Spitze der Elektrode 3 verbunden. Ferner ist ein Schalter C mit einem hochohmigen Parallelwiderstand 56 vorgesehen, die das Kontaktteil 51a über den Ausgangskondensator 55 mit dem Herzschrittmachergehäuse 2 verbinden. Die Detektoreinrichtung 43 weist eingangseitig ein Bandpaßfiler 61 auf, das über ein drittes Schalterpaar D und einen Eingangskondensator 62 mit den beiden Kontakteilen 51a, 51b der Elektrode 3 verbunden ist. Der Eingang des Bandpaßfilters 61 kann durch einen weiteren Schalter E kurzgeschlossen werden. Der weitere Schalter E wird ebenso wie der Schalter C und die Schalterpaare A,B und D über die ihne zugeordneten Steuerleitungen 22, 50 von dem Mikroprozessor 5 angesteuert. Das Bandpaßfilter 61 kann neben seiner Filterfunktion auch eine Verstärkerfunktion zur Signalverstärkung enthalten. Die Übertragungsfunktion des Bandpaßfilters 61 ist derart gewählt daß nur diejenigen Signalanteile, die hinsichtlich ihrer Frequenz bzw. Steilheit für stimulierte Herzmuskelkontraktionen typisch sind, das Bandpaßfilter 61 passieren können. Das Ausganssignal des Bandpaßfilters 61 gelangt zu dem nichtinvertierenden Eingang (+) eines Komparator 63, der dessen Amplitude mit einem Schwellwertsignal vergleicht, das seinem invertierenden Eingang (-) über die Steuerleitung 50 zugeführt ist. Bei dem Schwellwertsignal handelt es sich um das Ausgangssignal der Digital-/Analog-Schnittstelle 49. Über- oder unterschreitet die Amplitude das Ausgangssignals des Bandpaßfilters 61 den Pegel des Schwellwertsignals, springt das Ausganssignal des Komparators 63 von seinem einen Extremwert auf seinen anderen Extremwert um.

Fig. 6 zeigt in einem Zeitdiagramm ein Beispiel für den von dem Stimulationsimpuls-Generator 20 erzeugten negativen Stimulationsimpuls 64 mit nachfolgendem Schnellentladeimpuls 65 und die zur Steuerung des Stimulationsimpuls-Generators 20 und der Detektoreinrichtung 43 von dem Mikroprozessor 5 an die Schalterpaare A,B und D sowie die Schalter C und E abgegebenen , hier entsprechend bezeichneten Steuersignale. Dabei bedeutet ein von Null abweichende Signalzustand, daß der zugeordnete Schalter geschlossen ist. Solange das Schalterpaar A geschlossen ist, wird der Ladekondensator 54 auf die von der Ladeschaltung 53 erzeugte Ausgangsspannung aufgeladen. Zur Erzeugnung des negativen Stimulationsimpulses 64 wird bei nunmehr geöffnetem Schalterpaar A der Schalter B für die Dauer von etwa 1ms geschlossen. Nach einer anschließenden kurzen Pause wird der Schalter C für etwa 4ms geschlossen, wodurch der Ausgangskondensator 55 schnellentladen wird. Während der Dauer des Stimulationsimpulskomplexes 64, 65 ist der Eingang des Bandpaßfilters 61 durch das Schalterpaar D von den Kontaktteilen 51a, 51b der Elektrode 3 entkoppelt und über den weiteren Schalter E darüberhinaus eine kurze Zeit länger kurzgeschlossen.

Zur Erläuterung der Detektion von Herzmuskelkontraktionen mittels der Detektoreinrichtung 43 sind in Fig. 7 oben der Verlauf einer zwischen den Kontaktteilen 51a, 51b, also bipolar gemessenen stimulierten Herzmuskelkontraktion 66 und der Verlauf einer spontanen, natürlichen Herzmuskelkontaktion 67 dargestellt. Darunter sind die entsprechenden Signalverläufe 68 bzw. 69 nach ihrer Filterung durch das Bandpaßfilter 61 dargestellt. Wie der Fig. 7 zu entnehmen ist, weist der Signalverlauf 66 für die stimullierte Herzmuskelkontraktion etwa 50ms nach der Stimulation eine schnelle positive Flanke auf, die eine sichere Identifizierung des Signalverlaufs 66 als Antwort des Herzens auf eine Stimulation ermöglicht. Die Identifizierung erfolgt in der Weise, daß des Schwellenwertkomparator 63 das bandpaßgefilterte Signal 68 auf das Überschreiten eines ersten positiven Schwellenwertes 70 überwacht. Die Verzögerung der positiven Signalflanke gegenüber dem Stimulationsimpuls 64 bringt es mit sich, daß zum Zeitpunkt des Auftretens der Signalflanke die durch die Stimulation im Herzgewebe hervorgerufenen Polarisationserscheinungen zu einem großen Teil bereits abgeklungen sind. Ferne wird der Einfluß der Polarisationserscheinungen auf das gemessene Signal dadurch verringert, daß die beiden Kontaktteile 51a 51b aufgrund ihres geringen Abstandes voneinander beide im Bereich des polarisierten Gewebes liegen, so daß zwischen den beiden Kontaktteilen 51a, 51b kein nennenswertes polarisationsbedingtes Signal auftritt. Schließlich wirkt das Bandpaßfilter 63 sperrend für die relativ langsam abklingenden Polarisationserscheinungen. Bei dem Signalverlauf 69 eines natürlichen Herzschlages ist die negative Signalflanke am schnellsten und daher zur Identifizierung des Signalverlaufs 69 am besten geeignet, indem der Schwellenwertkomparator 63 das bandpaßgefilterte Signal 69 auf Unterschreiten eines zweiten, negativen Schwellenwertes 71 überwacht.

Die bereits erwähnte selbsttätige Einstellung des Energiegehaltes der mittels des Stimulationsimpuls-Generators 20 erzeugten Stimulationsimpulse erfolgt bei allen drei beschriebenen Ausführungsbeispielen derart, daß der Mikroprozessor 5 jedesmal nach Abgabe eines Stimulationsimpulses prüft, ob von dem Ausgang der Detektoreinrichtung 43 über die Leitung 46 ein die Detektion einer stimulierten Herzmuskelkontraktion anzeigendes Signal ankommt. Ist dies nicht der Fall, erhöht der Mikroprozessor 5 den Energiegehalt des nächsten Stimulationsimpulses, indem er der Digital-/Analog-Schnittstelle 24 digitale Daten zuführt, die diese in ein analoges Signal umsetzt, das den Stimulationsimpuls-Generator 20 derart einstellt, daß dieser einen Stimulationsimpuls mit einem gegenüber dem zuvor vorhanden Energiegehalt um einen definierten Schritt höheren Energiegehalt abgibt. Dies erfolgt so lange, bis eine Einstellung für den Energiegehalt der Stimulationsimpulse gefunden ist, bei der die Detektoreinrichtung 43 nach jedem Stimulationsimpuls eine stimulierte Herzmuskelkontraktion detektiert. Dabei stellt der Mikroprozessor 5 über die Digital/Analog-Schnittstelle 24 den Stimulationsimpuls-Generator 20 derart ein, daß der Energiegehalt der erzeugten Stimulationsimpulse der Summe aus einem MindestEnergiegehalt, bei dem die Detektoreinrichtung 43 nach der Abgabe eines Stimulationsimpulses gerade noch eine stimulierte Herzmuskelkontraktion detektiert, und einem Sicherheitsmarginal, z.B. 50% des Mindest-Energiegehaltes, entspricht. Dabei entspricht der Mindest-Energiegehalt der sogenannten Reizschwelle, die der Energiegehalt eines Stimulationsimpulses wenigstens erreichen muß, um eine stimulierte Herzmuskelkontraktion auslösen zu können. Zur Ermittlung des Mindest-Energiegehaltes senkt der Mikroprozessor 5 von Zeit zu Zeit den Energiegehalt der Stimulationsimpulse ausgehend von einem Wert, bei dem die Detektoreinrichtung 43 nach jedem Stimulationsimpuls einer Folge von Stimulationsimpulsen eine stimulierte Herzmuskelkontraktion detektiert, schrittweise allmählich so weit ab, bis wenigstens nach einzelnen Stimulationsimpulsen mittels der Detektoreinrichtung 43 keine stimulierte Herzmuskelkontraktion mehr detektierbar ist. Ausgehend von diesem Energiegehalt der Stimulationsimpulse erhöht der Mikroprozessor 5 deren Energiegehalt schrittweise allmählich wieder, und zwar gerade so weit, bis die Detektoreinrichtung 43 nach jedem Stimulationsimpuls wieder eine stimulierte Herzmuskelkontraktion detektiert. Der so gefundene Wert stellt den Mindest-Energiegehalt der Stimulationsimpulse bzw. die Reizschwelle dar.

Durch die beschriebene Einstellung des Energiegehaltes der Stimulationsimpulse wird erreicht, daß einerseits die Sicherheit des Patienten gewährleistet ist, da die Stimulation stets mit Stimulationsimpulsen erfolgt, deren Energiegehalt um ein Sicherheitsmarginal oberhalb des Mindest-Energiegehalltes liegt. Andererseits ist sichergestellt, daß der Energiebedarf des Gerätes durch die Abgabe der Stimulationsimpulse nicht höher als nötig ist, da sich deren Energiegehalt stets an dem erforderlichen Mindest-Energiegehalt orientiert.

## Patentansprüche

1. In den Körper eines Lebewesens inplantierbares medizinisches Gerät (1) mit Mitteln (20) zum elektrischen Stimulieren von Gewebekontraktionen, die einen ein Stimulationspotential und einen ein Bezugspotential führenden Anschluß aufweisen, mit Mitteln (43) zum Detektieren stimulierter Gewebekontraktionen, die eingangsseitig zwei Anschlüsse aufweisen und ein dem Potentialunterschied zwischen den beiden Auschlüssen entsprechendes Signal bilden, mit Mitteln (2) zum Anlegen des Bezugspotentials an den Körper des Lebewesens und mit einer bipolaren Elektrode (3) die zwei mit dem zu stimulierenden Gewebe in Kontakt bringbare Kontaktteile (51a, 51b) aufweist, wobei zur Stimulation einer Gewebekontraktion der das Stimulationspotential führende Anschluß der Mittel (20) zum Stimulieren mit wenigstens einem der beiden Kontaktteile (51a, 51b) der Elektrode (3) und der das Bezugspotential führende Anschluß der Mittel (20) zum Stimulieren mit den Mitteln (2) zum Anlegen des Bezungspotentials verbunden ist und nach erfolgter Stimulation einer der Anschlüsse der Mittel (43) zum Detektieren stimulierter Gewebekontraktionen mit einem der beiden Kontaktteile (51a, 51b) der Elektrode (3) verbunden ist, **dadurch gekennzeichnet,** daß nach erfolgter Stimulation der andere Anschluß der Mittel (43) zum Detektieren stimulierter Gewebekontraktionen mit dem anderen Kontaktteil (51) der Elektrode (3) verbunden ist.

2. Gerät nach Anspruch 1, **dadurch gekennzeichnet**, daß zur Stimulation einer Gewebekontraktion der das Stimulationspotential führende Anschluß der Mittel (20) zum Stimulieren mit nur einem Kontaktteil (51a) der Elektrode (3) verbunden ist, und daß der Abstand zwischen den Kontaktteilen (51a, 51b) der Elektrode (3) höchstens fünf Millimeter beträgt.

3. Gerät nach Anspruch 1, **dadurch gekennzeichnet**, daß Schaltmittel (59) vorgesehen sind, die zur Stimulation einer Gewebekontraktion den das Stimulationspotential führenden Anschluß der Mittel (20) zum Stimulieren mit beiden Kontaktteilen (51a, 51b) der Elektrode (3) verbinden.

4. Gerät nach einem der Ansprüche 1 bis 3, **dadurch gekennzeichnet**, daß die Mittel (43) zum Detektieren stimulierter Gewebekontraktionen einen Bandpaßfilter (61) mit einem nachgeordneten Schwellenwertdetektor (63) enthalten.

5. Gerät nach einem der Ansprüche 1 bis 4, **dadurch** **gekennzeichnet**, daß Mittel (27, 43) zum Detektieren spontaner Gewebekontraktionen vorgesehen und mit einer Steuerlogik (5) verbunden sind, die die Mittel (20) zum Stimulieren jeweils dann aktiviert, wenn mittels der Mittel (27, 43) zum Detektieren spontaner Gewebekontraktionen vor Ablauf eines definierten Zeitintervalls keine spontane Gewebekontraktion detektierbar ist.

6. Gerät nach Anspruch 5, **dadurch gekennzeichnet**, daß die Mittel zum Detektieren spontaner Gewebekontraktionen durch die Mittel (43) zum Detektieren stimulierter Gewebekontraktionen gebildet sind und daß Mittel (49) zum Einstellen unterschiedlicher Empfindlichkeiten der Mittel (43) zum Detektieren stimulierter bzw. spontanter Gewebekontraktionen vorgesehen sind.

7. Gerät nach Anspruch 6, **dadurch gekennzeichnet**, daß Schaltmittel (57) vorgesehen sind, mittels derer zum Detektieren spontaner Gewebekontraktionen ein Anschluß der Mittel (43) zum Detektieren stimulierter Gewebekontraktionen mit den Mitteln (2) zum Anlegen des Bezugspotentials verbindbar ist.

8. Gerät nach Anspruch 6, **dadurch gekennzeichnet**, daß die Mittel (43) zum Detektieren stimulierter und spontaner Gewebekontraktionen eingangsseitig fest mit den beiden Kontaktteilen (51a, 51b) der Elektrode (3) verbunden sind.

9. Gerät nach Anspruch 8, **dadurch gekennzeichnet**, daß die Mittel (20) zum elektrischen Stimulieren von Gewebekontraktionen ein gegenüber dem Bezugspotential negatives Stimulationspotential erzeugen, daß nach einer erfolgten Stimulation während eines ersten Zeitintervalls zur Detektion stimulierter Gewebekontraktionen (66) die Mittel (49) zum Einstellen unterschiedlicher Detektionsempfindlichkeiten einen ersten Schwellenwert (70) an dem Schwellenwertdetektor (63) einstellen, der das bandpaßgefilterte Signal (68) auf ein Überschreiten des ersten Schwellenwerts (70) überwacht, und daß in einem nachfolgenden zweiten Zeitintervall zur Detektion spontaner Gewebekontraktionen (67) die Mittel (49) zum Einstellen unterschiedlicher Detektionsempfindlichkeiten einen zweiten Schwellenwert (71) an dem Schwellenwertdetektor (63) einstellen, der das bandpaßgefilterte Signal (69) auf ein Unterschreiten des zweiten Schwellenwertes (71) überwacht.

## Claims

1. Medical appliance (1) which can be implanted into the body of a living organism and which has means (20) for the electrical stimulation of tissue contractions, which means exhibit a connection carrying a stimulation potential and a connection carrying a reference potential, having means (43) for detecting stimulated tissue contractions, which means exhibit on the input side two connections and form a signal corresponding to the potential difference between the two connections, having means (2) for applying the reference potential to the body of the living organism and having a bipolar electrode (3) which exhibits two contact parts (51a, 51b) which can be brought into contact with the tissue to be stimulated, in which in order to stimulate a tissue contraction that connection of the stimulating means (20) which carries the stimulation potential is connected to at least one of the two contact parts (51a, 51b) of the electrode (3) and that connection of the stimulating means (20) which carries the reference potential is connected to the means (2) for applying the reference potential and, after stimulation has taken place, one of the connections of the means (43) for detecting stimulated tissue contractions is connected to one of the two contact parts (51a, 51b) of the electrode (3), characterized in that, after stimulation has taken place, the other connection of the means (43) for detecting stimulated tissue contractions is connected to the other contact part (51) of the electrode (3).

2. Appliance according to Claim 1, characterized in that, in order to stimulate a tissue contraction, that connection of the stimulating means (20) which carries the stimulation potential is connected to only one contact part (51a) of the electrode (3), and in that the spacing between the contact parts (51a, 51b) of the electrode (3) is at most five millimetres.

3. Appliance according to Claim 1, characterized in that switching means (59) are provided, which, in order to stimulate a tissue contraction, connect that connection of the stimulating means (20) which carries the stimulation potential to both contact parts (51a, 51b) of the electrode (3).

4. Appliance according to one of Claims 1 to 3, characterized in that the means (43) for detecting stimulated tissue contractions include a band-pass filter (61) with a downstream threshold detector (63).

5. Appliance according to one of Claims 1 to 4, characterized in that means (27, 43) for detecting spontaneous tissue contractions are provided and are connected to a control logic circuit (5), which in each instance activates the stimulating means (20) when no spontaneous tissue contraction is detectable by means of the means (27, 43) for detecting spontaneous tissue contractions prior to the expiry of a defined time interval.

6. Appliance according to Claim 5, characterized in that the means for detecting spontaneous tissue contractions are formed by the means (43) for detecting stimulated tissue contractions, and in that means (49) are provided for setting differing sensitivities of the means (43) for detecting stimulated or spontaneous tissue contractions.

7. Appliance according to Claim 6, characterized in that switching means (57) are provided, by means of which, in order to detect spontaneous tissue contractions a connection of the means (43) for detecting stimulated tissue contractions is connectable to the means (2) for applying the reference potential.

8. Appliance according to Claim 6, characterized in that the means (43) for detecting stimulated and spontaneous tissue contractions are permanently connected, on the input side, to the two contact parts (51a, 51b) of the electrode (3).

9. Appliance according to Claim 8, characterized in that the means (20) for the electrical stimulation of tissue contractions generate a stimulation potential which is negative in relation to the reference potential, in that, after a stimulation has taken place during a first time interval for the detection of stimulated tissue contractions (66) the means (49) for setting differing detection sensitivities set a first threshold value (70) on the threshold value detector (63), which monitors the band-pass-filtered signal (68) for an exceeding of the first threshold value (70), and in that in a subsequent second time interval for the detection of spontaneous tissue contractions (67), the means (49) for setting differing detection sensitivities set a second threshold value (71) on the threshold value detector (63), which monitors the band-pass-filtered signal (69) for the presence of a value falling below the second threshold value (71).

## Revendications

1. Appareil médical (1) implantable dans le corps d'un être vivant et comportant des moyens (20) pour stimuler électriquement des contractions tissulaires et qui comportent une borne appliquant un potentiel de stimulation et une borne appliquant un potentiel de référence, des moyens (43) pour détecter les contractions tissulaires stimulées et qui comportent deux bornes côté entrée et forment un signal qui correspond à la différence de potentiel entre les deux bornes, des moyens (2) pour appliquer le potentiel de référence au corps de l'être vivant et une électrode bipolaire (3), qui comporte deux éléments de contact (51a, 51b) pouvant être amenés en contact avec le tissu à stimuler, pour stimuler une contraction tissulaire, et dans lequel la borne qui applique le potentiel de stimulation des moyens de stimulation (20), est raccordée à au moins l'un des deux éléments de contact (51a, 51b) de l'électrode (3), e la borne, qui applique le potentiel de référence des moyens de stimulation (20) est raccordée aux moyens (2) d'application du potentiel de référence et, après une stimulation exécutée de l'une des bornes des moyens (43) qui servent à détecter des contractions tissulaires stimulées, est connectée à l'un des deux éléments des contact (51a, 51b) de l'électrode (3), caractérisé par le fait qu'une fois exécutée la stimulation, l'autre borne des moyens (43) servant à détecter des contractions tissulaires stimulées est connectée à l'autre élément de contact (51) de l'électrode (3).

2. Appareil suivant la revendication 1, caractérisé par le fait que pour la stimulation d'une contraction tissulaire, la borne qui applique le potentiel de stimulation des moyens de stimulation (20), est connectée à seulement un élément de contact (51a) de l'électrode (3), et que la distance entre les éléments de contact (51a, 51b) de l'électrode (3) est égale au maximum à cinq millimètres.

3. Appareil suivant la revendication 1, caractérisé par le fait qu'il est prévu des moyens de commutation (59), qui, pour la stimulation d'une contraction tissulaire, raccordent la borne qui applique le potentiel de stimulation des moyens de stimulation (20), aus deux éléments de contact (51a, 51b) de l'électrode (3).

4. Appareil suivant l'une des revendications 1 à 3, caractérisé par le fait que les moyens (43) servant à détecter les contractions stimulées comportent un filtre passe-band (61), en aval duquel est branché un détecteur valeur de seuil (63).

5. Appareil suivant l'une des revendications 1 à 4, caractérisé par le fait qu'il est prévu des moyens (27, 43) pour détecter des contractions tissulaires spontanées et qui sont raccordés à une unité logique de commande (5), qui active les moyens de stimulation (20) chaque fois qu'aucune contraction tissulaire spontanée ne peut être détectée à l'aide des moyens (27, 43) de détection de contractions tissulaires spontanées, avant l'écoulement d'un intervalle de temps défini.

6. Appareil suivant la revendication 5, caractérisé en ce que les moyens pour détecter des contractions tissulaires spontanées sont formés par les moyens (43) servant à détecter les contractions tissulaires stimulées et qu'il est prévu des moyens (49) pour régler différentes sensibilités des moyens (43) servant à détecter des contractions tissulaires stimulées ou spontanées.

7. Appareil suivant la revendication 6, caractérisé par le fait qu'il est prévu des moyens de commutation (57), à l'aide desquels une borne des moyens (43) servant à détecter des contractions tissulaires stimulées peut être connectée, pour la détection de contractions tissulaires spontanées, aux moyens (2) servant à appliquer le potentiel de référence.

8. Appareil suivant la revendication 6, caractérisé par le fait que les moyens (43) servant à détecter les contractions tissulaires stimulées spontanées sont raccordés de facon fixe, côté entrée, aux deux éléments de contact (51a, 51b) de l'électrode (3).

9. Appareil suivant la revendication 8, caractérisé par le fait que les moyens (20) servant à stimuler électriquement des contractions tissulaires produisent un potentiel de stimulation négatif par rapport au potentiel de référence, qu'après une stimulation réalisée, les moyens (49) servant à régler différentes sensibilités de détection règlent, pendant un premier intervalle de temps, pour la détection de contractions tissulaires stimulées (66), une première valeur de seuil (70) dans le détecteur à valeur de seuil (63), qui surveille le signal (68) ayant subi le filtrage passe-bande pour voir s'il dépasse la première valeur de seuil (70), et qu'au cours d'un second intervalle de temps suivant, pour la détection de contractions tissulaires spontanées (67), les moyens (49) servant à régler différentes sensibilités de détection règlent une second valeur de seuil (71) dans le détecteur à valeur de seuil (63), qui surveille le signal (69) ayant subi le filtrage passe-band pour déterminer si ce signal est inférieur à la seconde valeur de seuil (71).
